# NEUE EUROPÄISCHE PATENTSCHRIFT

(11) **EP 0 182 955 B2**
(45) Veröffentlichungstag und Bekanntmachung des Hinweises auf die Entscheidung über den Einspruch: **25.06.1997**
(45) Hinweis auf die Patenterteilung: 04.01.1989
(21) Anmeldenummer: 85100494.5
(22) Anmeldetag: 18.01.1985
(51) Int. Cl.: C02F 3/28

(54) **Reaktionsgefäss**
Reaction vessel
Cuve de réaction

(30) Priorität: 20.11.1984 CH 5531/84
(43) Veröffentlichungstag der Anmeldung: 04.06.1986
(73) Patentinhaber: GEBRÜDER SULZER AKTIENGESELLSCHAFT, CH-8401 Winterthur (CH)
(72) Erfinder: Lührs, Hermann, Dr., D-4048 Grevenbroich 2 (DE); Preetz, Jürgen, Dipl.-Ing., D-6366 Wölfersheim (DE); Redwanz, Jürgen, D-4040 Neuss (DE)
(74) Vertreter: Heubeck, Bernhard

(56) Entgegenhaltungen:
- EP-A- 0 058 247
- EP-A- 0 172 443
- CH-A- 634 803
- DE-A- 2 554 495
- DE-C- 907 280
- DE-U- 8 326 116
- GB-A- 1 491 502
- US-A- 2 605 220
- Veröffentlichungen des Institutes für Siedlungswasserwirtschaft der Universität Hannover, Heft 50, 1979, Seiten 234-237 (Sixt)
- Zuckerindustrie 107 (1982), Nr. 9, Seiten 835-838 (Hasenböhler)
- Zuckerindustrie 109 (1982), Nr. 1, Seiten 28-33 (Sixt)

## Beschreibung

Die Erfindung betrifft ein Reaktionsgefäss für einen Methan-Reaktor gemäss Oberbegriff von Anspruch 1.

Ein Reaktionsgefäss der vorstehend genannten Art ist aus einer in dem Dokument CH-A-634 803 beschriebenen Anlage bekannt. Diese Anlage enthält ein Fermentationsabteil, ein zylindrisches Sedimentierabteil, ein Rohr und eine Überlaufrinne, die an der zylindrischen Wandung des Fermentierabteils angeordnet ist. Das aus dem Fermentationsabteil verdrängte Schlamm-Wasser-Gemisch fliesst durch das Rohr in das Sedimentierabteil, wo die Reinflüssigkeit durch einfache Sedimentation vom Schlamm getrennt wird. In der bekannten Anlage ist ein Flockenbildner oder Separator vorgesehen, mit dem ein rascheres Absetzen des Schlamms im Sedimentierabteil erzielt wird. Das Schlamm/Wasser-Gemisch wird erst nach Austritt aus dem Flockenbildner in einem voluminös ausgebildeten Teil des Sedimentierabteils getrennt.

Es ist Aufgabe der Erfindung, ein Reaktionsgefäss für einen Methan-Reaktor so zu verbessern, dass eine kompaktere Baueinheit entsteht. Gelöst wird diese Aufgabe durch das in Anspruch 1 definierte Reaktionsgefäss.

Mit Vorteil wird die Einspeisevorrichtung so ausgebildet, dass in dem Reaktionsraum ein zentrales, mit einer Umwälzeinrichtung versehenes Zulauf- bzw. Zirkulationsrohr für die zu behandelnde bzw. für die mit Biomasse durchsetzte Flüssigkeit eingebaut wird.

Zur Überwachung der Schrägklärer ist es vorteilhaft, wenn der diese aufnehmende Sedimentierraum, der nachfolgend als Teilraum bezeichnet wird nach oben offen und/oder wenn der Raum über dem Teilraum begehbar ist. Eine schonende Rückführung der abgetrennten Biomasse in den Reaktionsraum lässt sich erreichen, wenn der Teilraum nach unten über trichterartige Schlammsammeltaschen gegen den Reaktionsraum abgeschlossen ist, von denen Schlammrückführöffnungen direkt in den Reaktionsraum führen.

Den "Antrieb" für eine Zirkulation der behandelten Flüssigkeit durch den oder die Schrägklärer bewirken im einfachsten Fall Dichtunterschiede im Reaktionsraum und in der Flüssigkeit, die durch die teilweise Entgasung des Gemisches durch die freie Niveaufläche des Reaktionsraumes hindurch und durch die Abscheidung der Feststoffe der Biomasse in den Schrägklärer entstehen. Sollte dieser Antrieb zu gering sein, so kann man zwischen dem Reaktionsraum und dem Schrägklärer mindestens eine Förder- und Verteileinrichtung für das im Reaktionsraum behandelte Gemisch aus Flüssigkeit und Biomasse vorsehen; eine solche Fördereinrichtung gibt dabei die Möglichkeit, die interne Zirkulation in dem Reaktionsgefäss zu dosieren und zu beherrschen. Zusätzlich kann darüberhinaus die Förderleistung der Fördereinrichtung, die mit Vorteil z. B. ein Flügelpropeller ist, - beispielsweise mit Hilfe einer Drehzahlregelung oder mit Hilfe von Drosselorganen im Ansaug der Fördereinrichtung - regel-regel- und/oder einstellbar sein, wodurch eine gleichmässige definierte Beschickung der Schrägklärer sichergestellt wird. Diese definierte Beschickung kann mit Vorteil zwischen der einfachen und der doppelten Menge des zufliessenden Rohflüssigkeitsstromes varieren.

Durch die Fördereinrichtung erfolgt darüberhinaus eine zusätzliche Entgasung der Flüssigkeit, bevor sie auf die Schrägklärer geführt wird; diese Entgasung kann intensiviert werden, wenn die Fördereinrichtung einen Schwimmschlammabscheide enthält, wobei für die mechanische Räumung des Abscheiders ein mit der Fördereinrichtung umlaufender Räumer, z. B. ein Krählwerk, vorgesehen sein kann.

Im folgenden wird die Erfindung anhand von Ausführungsbeispielen im Zusammenhang mit den Zeichnungen näher erläutert.
Fig. 1 zeigt in einem Schnitt I-I von Fig. 2 einen axialen Längsschnitt durch eine erste Ausführungsform des neuen Reaktionsgefässes;
Fig. 2 ist ein Aufsicht auf Fig. 1 von oben, wobei der den Gasraum des Behälters abschliessende Deckel abgenommen ist;

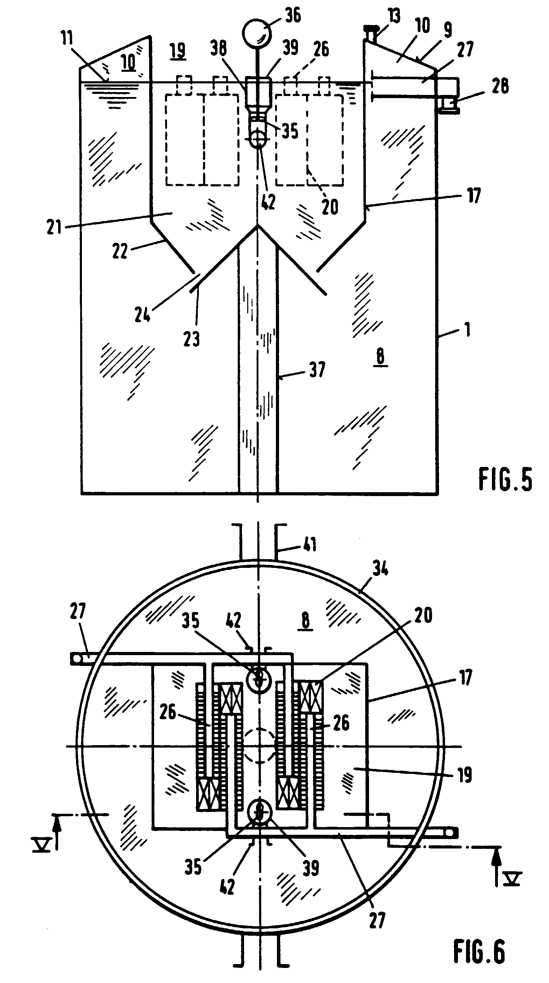

Fig. 3 und 4 bzw. Fig 5 und 6 stellen in gleicher Darstellungsweise wie Fig. 1 und 2 ein zweites bzw. ein drittes Ausführungsbeispiel dar, während
Fig. 7 in vergrössertem Maßstab als Detail aus Fig. 1 den Aufbau einer Fördereinrichtung für die interne Zirkulation wiedergibt.

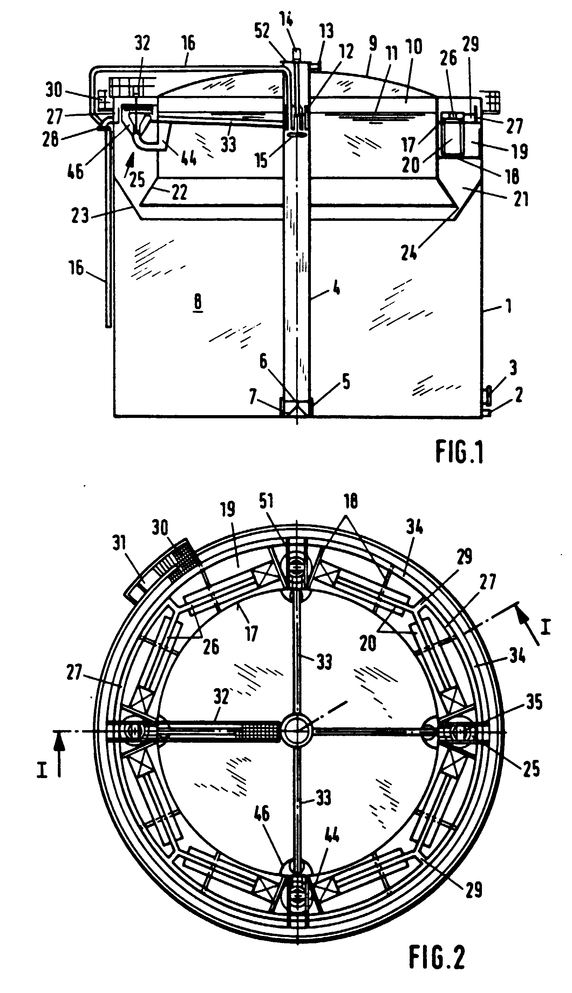

Das Reaktionsgefäss besteht aus einem kreiszylindrischen Behälter 1, beispielsweise aus Stahlblech, den an seinem oberen Rand eine Randverstärkung 34 (Fig. 2) umschliesst. In Fig. 1 ist in seinem Mantel ein Schlammabzugsstutzen 2 und ein Mannloch 3 angedeutet.

Der Behälter 1 enthält einen Reaktionsraum 8; in ihn sind darüberhinaus die Mittel integriert, durch die - wie später noch beschrieben wird - das im Behälter 1 behandelte Gemisch in Reinflüssigkeit, Biomasse und gasförmige Reaktionsprodukte, vor allem Methan und Kohlendioxid, separiert wird.

Im Zentrum des Behälters 1 ist ein zentrales Zulauf- und Zirkulationsrohr 4 vorgesehen, das kurz über dem Boden des Behälters 1 endet und in einem fussartigen Gerüst 5 gehalten ist, so dass in Bodennähe Austrittsöffnungen 6 für die zugeführte bzw. umgewälzte Flüssigkeit verbleiben, in denen zusätzlich eine leitblechartige, z. B. kegelförmige, Schikane 7 vorgesehen ist. Das Zulaufrohr 4 enthält in der Höhe des den Reaktionsraum 8 von einem darüberliegenden mit einem Deckel 9 gegen die Umgebung abgeschlossenen Gasraum 10 trennenden Flüssigkeitsniveaus auf seinem Umfang verteilt Durchtrittsöffnungen 12 für die umzuwälzende Flüssigkeit. Diese Öffnungen 12 ragen teilweise in den Gasraum 10 hinein, so dass die Reaktionsgase durch den oberen Teil des Rohres 4 hindurch über einen Gasabzug 13 aus dem Behälter 1 entfernt werden können.

In den gasdichten Deckel 52 des Zulaufrohres 4 kann von oben ein durch einen Motor 14 angetriebener Rührer 15 zur Förderung und Umwälzung der Flüssigkeit in und durch das Rohr 4 eingesetzt werden; weiterhin mündet in das Rohr 4 eine Speiseleitung 16 für die zu behandelnde Rohflüssigkeit.

Mit Abstand vom Mantel des Behälters 1 ist im Bereich des Flüssigkeitsniveaus 11 eine Zwischenwand 17 vorgesehen, die bei der ersten Ausführungsform einen mit dem Rohr 4 und dem Behälter 1 konzentrischen Ring bildet; durch diese Zwischenwand 17, die von einer Anzahl Streben 18 gehalten ist und gleichzeitig als Auflage für den Deckel 9 dient, wird ein ringförmiger Teilraum 19 vom Reaktionsraum 8 abgetrennt. In diesem Teilraum 19 sind auf den Streben 18 gelagert als Mittel zur Abscheidung der Biomasse von der Flüssigkeit auf dem ganzen Umfang Schrägklärer 20 verteilt. Nach unten ist der ringförmige Teilraum 19 durch von zwei schrägen Blechen 22 und 23 gebildete trichterartige Schlammsammeltaschen 21 abgeschlossen, aus denen ein Schlammdurchtrittsschlitz 24 als Schlammrückführöffnung in den Reaktionsraum 8 führt.

Zur Speisung mit dem zu trennenden Gemisch sind Förder- und Verteileinrichtungen 25 zwischen den Schrägklärern 20 verteilt; ihr genauer Aufbau wird später beschrieben. Der Abzug der abgetrennten Reinflüssigkeit erfolgt mit Hilfe von auf den Schrägklärern 20 angeordneten Sammelrinnen 26, die über kleine Querrinnen 29 in eine zirkulare Abzugsrinne 27 münden. An dieser ist ein Auslaufstutzen 28 zum Anschluss einer Auslaufleitung für die Reinflüssigkeit vorgesehen.

Der ringförmige Teilraum 19 des Behälters 1 ist nach oben offen und über einen nur andeutungsweise wiedergegebenen peripheren Gittersteg 30 begehbar, der an einer Stelle des Umfangs mit einem Aufgang 31 verbunden ist. Zur Wartung und Überwachung des Motors 14 und des Rührers 15 führt ein weiterer Gittersteg 32, der ebenfalls nur skizziert ist, radial in das Zentrum des Behälters 1.

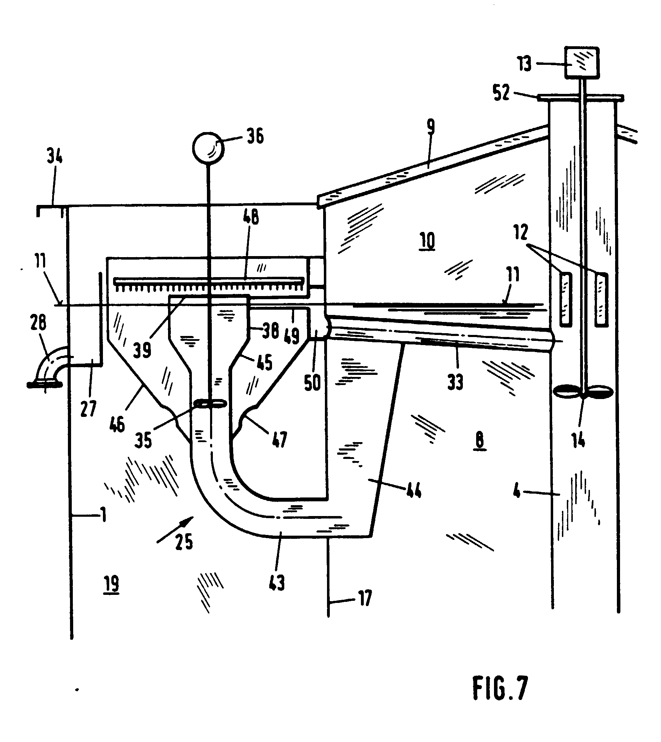

Schliesslich sind in Fig. 1 und 2 noch Rückströmleitungen 33 wiedergegeben, die von jeder Fördereinrichtung 25 leicht nach unten geneigt zum Zentralrohr 4 führen und den Rücktransport von in der Fördereinrichtung 25 abgetrenntem Schwimmschlamm dienen, wie in Zusammenhang mit Fig. 7 noch erläutert wird.

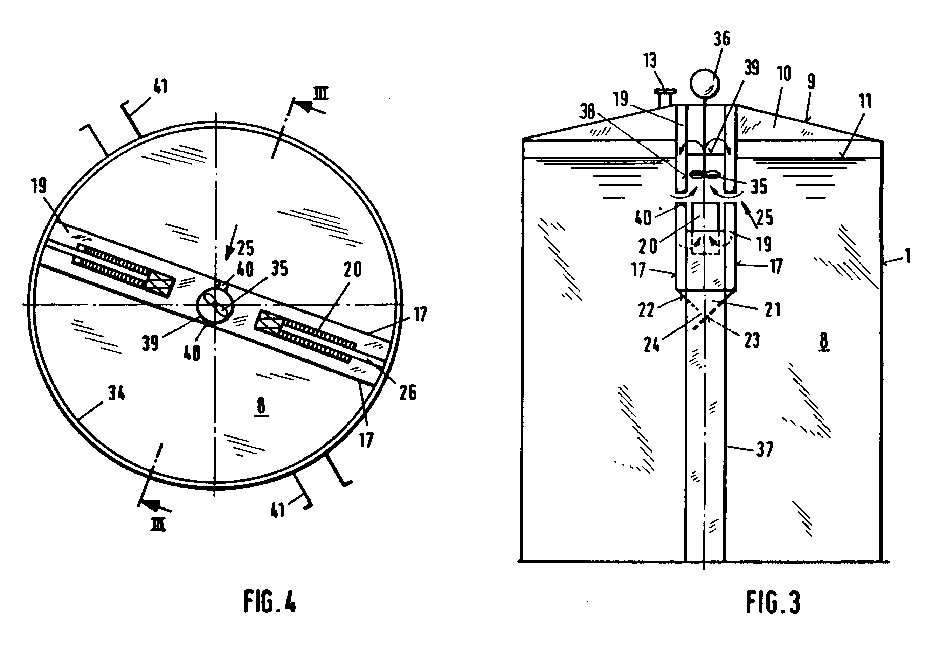

In dem stark schematisiert dargestellten Ausführungsbeispiel nach Fig. 3 und 4, in dem für funktionell gleichartige Elemente die in Fig. 1 und 2 benutzten Bezugsziffern verwendet sind, schneiden zwei über dem Durchmesser des Behälters 1 verlaufende Zwischenwände 17 einen scheibenartigen Teilraum 19 im Zentralbereich des Behälters 1 aus dem Reaktionsraum 8 aus. In diesem Teilraum 19, der wiederum oben offen und begehbar sein kann, was nicht nochmals ausdrücklich gezeigt ist, sind in radialer Richtung zwei Schrägklärer 20 angeordnet. Zwischen diesen liegt im Zentrum eine Fördereinrichtung 25, die einen als Flügelpropeller ausgebildeten Rührer 35 enthält, der von einem Motor 36 angetrieben ist. Die Fördereinrichtung 25 ruht auf einer Mittelsäule 37, die in einen den Rührer 35 umschliessenden Zylinder 38 übergeht, der in einer Überlaufkante 39 endet. Der Innenraum des Zylinders 38 steht unterhalb des Rührers 35 durch Rohrstutzen 40 mit dem Reaktionsraum 8 in Strömungsverbindung. Durch diese Rohrstutzen 40 saugt der Rührer 35 behandeltes, zu trennendes Gemisch an und fördert es über die Kante 39 in den Teilraum 19, aus dem es - wie die Pfeile andeuten - von unten in die Schrägklärer 20 zur Abscheidung der Biomasse eintritt.

Die Einspeisung der zu behandelnden Rohflüssigkeit in den Behälter 1 erfolgt über zwei in Fig. 4 schematisch angedeutete Zufuhrleitungen 41, die vorzugsweise in der Nähe des Behälterbodens angeordnet sind.

Bei der wiederum stark schematisch wiedergegebenen dritten Ausführungsform trennt die Zwischenwand 17 zentral einen quaderförmigen Teilraum 19 vom Reaktionsraum 8 ab. In den Fördereinrichtungen 25 sind auch bei diesem Beispiel zwei Rührer 35 vorgesehen, die saugseitig über die Wand 17 durchtretende Rohrbogen 42 mit dem Reaktionsraum 8 in Verbindung stehen. Auch bei dieser Ausführungsform wird das von den Rührern 35 geförderte, zu trennende Gemisch über Überlaufkanten 39 auf die Schrägklärer 20 verteilt.

Abschliessend seien anhand von Fig. 7, die einen vergrösserten Ausschnitt von Fig. 1 dargestellt, der detaillierte Aufbau und die Wirkungsweise der Fördereinrichtung 25 nach Fig. 1 näher erläutert. Wie die beiden vorstehend beschriebenen Fördereinrichtungen enthält die Fördereinrichtung 25 nach Fig. 1 einen Flügelpropeller 35, der von einem Motor 36 angetrieben ist. Auf der Saugseite des Rührers 35 führt ein Rohrbogen 43 durch die Zwischenwand 17 hindurch zu einem Ansaugtrichter 44, der im Flüssigkeitsinhalt des Reaktionsraums 8 unter das Niveau 11 getaucht angeordnet und daher mit in dem Reaktionsraum 8 behandelten zu trennendem Gemisch von Flüssigkeit und Biomasse gefüllt ist.

Auf der Förderseite schliesst an den Rührer 35 ein Trichter 45 an, der wiederum in einen Zylinder 38 mit einer Überlaufkante 39 übergeht. Der Zylinder 38 ist konzentrisch umschlossen von einem ebenfalls trichterförmigen Schwimmschlammabscheider 46, in dessen Trichter Öffnungen 47 für den Durchtritt des geförderten Gemisches in den Teilraum 19 und damit in die Schrägklärer 20 vorgesehen sind. Der Abscheider 46 dient einerseits zur Beruhigung des vom Rührer 35 geförderten Mengenstromes und andererseits zu einer Abscheidung von Schwimmschlamm, wobei gleichzeitig eine zusätzliche Entgasung des Gemisches - in dem gezeigten Beispiel in die freie Atmosphäre hinein - bewirkt wird. Schlammabscheidung und Entgasung werden unterstützt und gefördert durch ein mit dem Rührer 35 umlaufendes Krählwerk 48. Für die Abführung des abgeschiedenen Schwimmschlammes dient eine Radialrinne 49, die in einen Schlammtopf 50 mündet, von dem die Leitung 33 für den Rücktransport des Schwimmschlammes in das Zulaufrohr 4 bzw. den Reaktionsraum 8 ausgeht.

Die ganze Fördereinrichtung 25 ist gelagert und gehalten auf bzw. an zwei schienenartigen Stützen 51 (Fig. 2), die in radialer Richtung zwischen Behälter 1 und Zwischenwand 17 ausgelegt sind.

## Patentansprüche

1. Reaktionsgefäss für einen Methan-Reaktor zur anaeroben Behandlung von verunreinigten Flüssigkeiten, insbesondere Abwässern, mit einem Reaktionsraum (8), der von einem gegen die Umgebung abgeschlossenen Gasraum (10) überlagert ist und einem Sedimentierraum (19), der vom Reaktionsraum (8) mittels mindestens einer im Bereich des Flüssigkeitsspiegels (11) angeordneten Wand (17) abgetrennt ist, wobei ein Schrägklärer (20), eine Überlaufrinne (27) und eine Schlammsammeltasche (21) im Sedimentierraum (19) angeordnet sind, wobei der Sedimentierraum in direkter Verbindung mit dem Reaktionsraum steht,
dadurch gekennzeichnet, dass eine Mehrzahl von Schrägklärern (20) innerhalb des Sedimentierraums (19) vorgesehen ist und dass ein Sammelrinnensystem (26, 29) auf den Schrägklärern (20) angeordnet ist, das mit der Überlaufrinne (27) flüssigkeitsübertragend verbunden sein kann, um die in den Schrägklärern abgetrennte Reinflüssigkeit direkt aus diesen abzuleiten.

2. Reaktionsgefäss nach Anspruch 1 dadurch gekennzeichnet, dass der Sedimentierraum(19) durch Wandungen gebildet ist, die zwei in gleichem Abstand nebeneinander verlaufende, über der Schlammsammeltasche angeordnete Wandabschnitte umfassen und die Schlammsammeltasche mittels zueinander geneigt verlaufender Wandabschnitte gebildet ist.

3. Reaktionsgefäss nach Anspruch 1, dadurch gekennzeichnet, dass der Reaktionsraum (8) ein zentrales, mit einer Umwälzeinrichtung (15) versehenes Zulauf- bzw. Zirkulationsrohr (4) für die zu reinigende bzw. für die mit Biomasse durchsetzte Flüssigkeit umgibt.

4. Reaktionsgefäss nach Anspruch 1, dadurch gekennzeichnet, dass derTeilraum (19) nach oben offen ist.

5. Reaktionsgefäss nach Anspruch 1, dadurch gekennzeichnet, dass der Raum über dem Teilraum (19) begehbar ist.

6. Reaktionsgefäss nach Anspruch 1, dadurch gekennzeichnet, dass der Teilraum (19) nach unten über trichterartige Schlammsammeltaschen (21) gegen den Reaktionsraum (8) abgeschlossen ist, von denen Schlammrückführöffnungen (24) direkt in den Reaktionsraum (8) führen.

7. Reaktionsgefäss nach Anspruch 1, dadurch gekennzeichnet, dass zwischen dem Reaktionsraum (8) und dem Schrägklärer (20) mindestens eine Förder- und Verteileinrichtung (25) für das im Reaktionsraum (8) behandelte Gemisch aus Flüssigkeit und Biomasse vorgesehen ist.

8. Reaktionsgefäss nach Anspruch 1 und 7, dadurch gekennzeichnet, dass die Zwischenwand (17) in Umfangsrichtung in sich geschlossenmit Abstand von der Gefässwand (1) angeordnet ist, und dass ferner die Schrägklärer (20) über den Umfang gleichmässig verteilt sind, wobei je zwei Schrägklärer (20) einer gemeinsamen Fördereinrichtung (25) zugeordnet sind.

9. Reaktionsgefäss nach Anspruch 7, dadurch gekennzeichnet, dass die Förderleistung der Fördereinrichtung (25) regel-und einstellbar ist.

10. Reaktionsgefäss nach Anspruch 7, dadurch gekennzeichnet, dass die Fördereinrichtung (25) einen Flügelpropeller (35) enthält.

11. Reaktionsgefäss nach Anspruch 7, dadurch gekennzeichnet, dass der Fördereinrichtung (25) ein Schwimmschlamm-Abscheider (46) nachgeordnet ist, von dem ein Rücklaufrohr (33) zum zentralen Zulaufrohr (4) des Gefässes führt.

12. Reaktionsgefäss nach Anspruch 11, dadurch gekennzeichnet, dass in der Fördereinrichtung (25) ein in dem Schwimmschlamm-Abscheider (46) umlaufender Räumer (40), z. B. ein Krählwerk, vorgesehen ist.

## Claims

1. A reaction vessel for a methane reactor for anaerobic treatment of contaminated liquids, more particularly waste water, comprising a reaction chamber (8) underneath a gas chamber (10) sealed from the environment, and a sedimentation chamber (19) separated from the reaction chamber (8) by means of a wall (17) disposed in the region of the liquid level (11), a baffle plate thickener (20), an overflow channel (27) and a sludge-collecting pocket (21) being disposed in the sedimentation chamber (19), the latter being directly connected to the reaction chamber,
characterised in that a plurality of baffle plate thickeners (20) are provided inside the sedimentation chamber (19) and in that a collecting channel system (26, 29) is disposed on the baffle plate thickeners (20) and can be connected to the overflow channel (27) for the purpose of transferring liquid in order to discharge directly from the baffle plate thickeners the clean liquid separated therein.

2. A reaction vessel according to claim 1,
characterised in that the sedimentation chamber (19) is formed by walls which comprise two wall portions which extend side by side in equidistant relationship above the sludge-collecting pocket and the latter is formed by wall portions extending at an inclination to one another.

3. A reaction vessel according to claim 1,
characterised in that the reaction chamber (8) surrounds a central inlet or circulation pipe (4) comprising a device (15) for circulating the liquid to be purified or containing biomass.

4. A reaction vessel according to claim 1,
characterised in that the compartment (19) is open at the top.

5. A reaction vessel according to claim 1,
characterised in that the space above the compartment (19) is accessible.

6. A reaction vessel according to claim 1,
characterised in that the compartment (19) is closed off at the bottom from the reaction chamber (8) by funnel-like sludge-collecting pockets (21), from which openings (24) for returning sludge extend directly into the reaction chamber (8).

7. A reaction vessel according to claim 1,
characterised in that at least one device (25) for conveying and distributing the mixture of liquid and biomass treated in the reaction chamber (8) is provided between the reaction chamber (8) and the baffle plate thickener (20).

8. A reaction vessel according to claims 1 and 7,
characterised in that in the peripheral direction the partition (17) is closed and spaced apart from the vessel wall (1), and the baffle plate thickeners (20) are distributed uniformly around the periphery, each pair of thickeners (20) being associated with a common conveying device (25).

9. A reaction vessel according to claim 7,
characterised in that the conveying capacity of the conveying device (25) is controllable and adjustable.

10. A reaction vessel according to claim 7,
characterised in that the conveying device (25) comprises an impeller (35).

11. A reaction vessel according to claim 7,
characterised in that the conveying device (25) is associated with a scum separator (46) from which a return pipe (33) leads to the central inlet pipe (4) of the vessel.

12. A reaction vessel according to claim 11,
characterised in that an evacuating device (40), e.g. a raking mechanism, rotating in the scum separator (46) is provided in the conveying device (25).

## Revendications

1. Cuve de réaction pour un réacteur à méthane en vue du traitement anaérobie de liquides souillés, en particulier d'eaux usées, comprenant un compartiment de réaction (8) surmonté par un compartiment de gaz (10) isolé de l'environnement, et comprenant un compartiment de sédimentation (19) qui est séparé du compartiment de réaction (8) par au moins une cloison (17) disposée dans la zone du niveau de liquide (11), un clarificateur à tôles inclinées (20), une rigole de trop-plein (27) et une poche collectrice de boue (21) étant prévus dans le compartiment de sédimentation (19), le compartiment de sédimentation communiquant directement avec le compartiment de réaction, caractérisée en ce qu'une pluralité de clarificateurs à tôles inclinées (20) est prévue à l'intérieur du compartiment de sédimentation (19), et en ce que, sur les clarificateurs à tôles inclinées (20), est disposé un réseau de rigoles collectrices (26, 29) qui peut être relié à la rigole de trop-plein (27) en transportant du liquide afin d'évacuer directement des clarificateurs à tôles inclinées le liquide pur qui y a été déversé.

2. Cuve de réaction selon la revendication 1, caractérisée en ce que le compartiment de sédimentation (19) est formé par des cloisons qui comportent deux portions de cloison disposées à égale distance côte à côte et placées au-dessus de la poche collectrice de boue, et la poche collectrice de boue est formée au moyen de portions de cloison inclinées les unes vers les autres.

3. Cuve de réaction selon la revendication 1, caractérisée en ce que le compartiment de réaction (8) entoure un tube central d'arrivée et de circulation (4) muni d'un dispositif de brassage (15) et destiné au liquide que l'on veut épurer ou qui est chargé de biomasse.

4. Cuve de réaction selon la revendication 1, caractérisée en ce que le compartiment partiel (19) est ouvert vers le haut.

5. Cuve de réaction selon la revendication 1, caractérisée en ce que l'espace situé au-dessus du compartiment partiel (19) est praticable.

6. Cuve de réaction selon la revendication 1, caractérisée en ce que, vers le bas, le compartiment partiel (19) est séparé du compartiment de réaction (8) par des poches collectrices de boue en forme d'entonnoirs (21) dont des ouvertures de retour de boue (24) débouchent directement dans le compartiment de réaction (8).

7. Cuve de réaction selon la revendication 1, caractérisée en ce que, entre le compartiment de réaction (8) et le clarificateur à tôles inclinées (20), est prévu au moins un dispositif de transport et de répartition (25) pour le mélange traité dans le compartiment de réaction (8) et constitué de liquide et de biomasse.

8. Cuve de réaction selon les revendications 1 et 7, caractérisée en ce que la paroi intermédiaire (17) fermée dans le sens circonférentiel est disposée à distance de la paroi (1) de la cuve, et en ce qu'en outre les clarificateurs à tôles inclinées (20) sont répartis uniformément sur la périphérie, deux clarificateurs à tôles inclinées (20) étant associés à un dispositif de transport commun (25).

9. Cuve de réaction selon la revendication 7, caractérisée en ce que la capacité de transport du dispositif de transport (25) est réglable et ajustable.

10. Cuve de réaction selon la revendication 7, caractérisée en ce que le dispositif de transport (25) comporte une hélice à pales (35).

11. Cuve de réaction selon la revendication 7, caractérisée en ce qu'à la suite du dispositif de transport (25) est implanté un séparateur de boue surnageante (46), à partir duquel un tuyau de retour (33) conduit au tube central d'arrivée (4) de la cuve.

12. Cuve de réaction selon la revendication 11, caractérisée en ce qu'une curette (40), par exemple un mécanisme de raclage, tournant dans le séparateur de boue surnageante (46), est prévue dans le dispositif de transport (25).
